# EUROPEAN PATENT SPECIFICATION

(11) **EP 0 413 587 B1**
(45) Date of publication and mention of the grant of the patent: **24.01.1996**
(21) Application number: 90309018.1
(22) Date of filing: 16.08.1990
(51) Int. Cl.: G01N 33/86

(54) **Method for direct chemical binding of D-dimer from a biological sample for diagnosing and monitoring thrombolytic and hypercoagulable states**
Verfahren für direkte chemische Bindung des D-Dimers aus einer biologischen Probe zwecks Diagnostik und Überwachung von thrombolytischen und hyperkoagulablen Zuständen
Méthode d'association chimique directe de dimère-D provenant d'un échantillon biologique et permettant le diagnostic et le suivi d'états de thrombolyse et d'hypercoagulabilité

(30) Priority: 17.08.1989 US 395446
(43) Date of publication of application: 20.02.1991
(60) Divisional application: 95200426.5
(73) Proprietor: ORTHO DIAGNOSTIC SYSTEMS INC., Raritan, New Jersey 08869-0602 (US)
(72) Inventor: Thomas, Karen Ann, Somerville, NJ 08876 (US); Warunek, David James, East Brunswick, NJ 08816 (US)
(74) Representative: Mercer, Christopher Paul

(56) References cited:
- EP-A- 0 122 478
- US-A- 4 090 846
- JOURNAL OF BIOCHEMICAL CHEMISTRY, vol. 254, no. 11, June 1979, US; S.A. OLEXA et al., pp. 4925-4932/

## Description

This invention relates to a method of detecting D-dimer, a fibrin breakdown product which can be indicative of disorders of the blood clotting system, which utilizes a novel Fragment E reagent, which directly chemically binds to D-dimer in a biological sample.

The most common disorder of the human blood clotting system is the formation of thrombi, blood clots, which when formed in vessels such as the heart can restrict blood flow and result in severe heart attacks. In addition, movement of the thrombus, or a portion of it within the circulatory system can result in thromboembolism, by suddenly blocking blood flow. Patients receiving fibrinolytic therapy due to hypercoagulable conditions require careful monitoring of clot breakdown. Therefore, it is important to have sensitive and specific means to detect fibrinolysis, the breakdown of blood clots.

Fibrin consists of a web of branching fibers of an adhesive nature, with the power of coagulating whole blood in which it is suspended. Fibrinolysis is a system complementary to the coagulation process, and normally in equilibrium with it, disposing of deposits of fibrin. The digestion of fibrin by the enzyme plasmin produces split products including D-dimer.

In pathologic fibrinolysis, plasma concentrations of plasmin degradation products of fibrinogen and fibrin are augmented. Therefore, the presence of fibrinogen breakdown products (FDP) in human blood or urine are indicative of thrombotic disorders. FDP have been measured traditionally by agglutination assays. For example: by staphylococcal clumping; by hemagglutination immunoassay; by the use of latex particles to which an antiserum has been attached.

Hawiger et al., "Measurement of Fibrinogen and Fibrin Degradation Products in Serum by Staphylococcal Clumping Test", J. Lab. Clin. Med. 75:93-108 (1970) disclosed a test based on the fact that a specific strain of Staphylococcus will clump in the presence of monomeric fibrin or higher molecular weight FDP. In this method, serial dilutions of serum or urine samples are mixed with suspensions of Staphylococcus cells and the presence or absence of clumping is noted.

Merskey et al., "A Rapid, Simple, Sensitive Method for Measuring Fibrinolytic Split Products in Human Serum", Proc. Soc. Exp. Biol. Med. 131:871-875 (1969) disclosed a two-step hemagglutination-inhibition test. In the first step, dilutions of the test serum sample are incubated with a dilute solution of antibodies to human fibrinogen. In the second step, unreacted antibody is detected by adding tanned red blood cells coated with human plasma. If no agglutination occurs, the sample contained sufficient FDP to neutralize the antibody.

Carney et al., "A Sensitive Latex Slide Assay for Fibrin(ogen) Fragment E", Clinical Chimica Acta 147:173-177 (1985), disclosed a latex agglutination test wherein latex particles were coated with antibodies which react directly with FDP in serum. The use of a monoclonal antibody DD-3B6/22 to D-dimer introduced the specificity required to differentiate between fibrin and fibrinogen breakdown products. This was an important improvement because detection of D-dimer, a fibrin breakdown product, is more specific for the presence of clots, which permits detection of thrombosis or hypercoagulable states with greater sensitivity and specificity than was possible with other techniques. As disclosed in an article by Elms et al., "Rapid Detection of Cross-Linked Fibrin Degradation Products in Plasma Using Monoclonal Antibody Coated Latex Particles", A.J.C.P. 85(3):360-364 (March 1986), detection of the conformational and structural changes on conversion of fibrinogen to fibrin and its cross-linking by Factor XIIIa in the process of clot formation, led to the development of new antigenic determinants that permit differentiation between their plasminolytic cleavage products. Specifically, a monoclonal antibody (DD-3B6/22) that is specific for cross-linked fibrin derivatives containing the D-dimer configuration was used in developing a latex agglutination procedure able to detect fibrin breakdown products in either plasma or serum. The sensitivity of this assay appears to be approximately 250 ng/ml of D-dimer.

Greenberg et al. in an article "Measurement of Plasma Fibrin D-dimer Levels with the Use of a Monoclonal Antibody Coupled to Latex Beads", A.J.C.P. 87(1) 94-100 (January 1987), disclosed a monoclonal antibody (DD-3B6) to fibrin D-dimer which was coupled to latex beads to provide a specific test (Dimertest) for fibrinolysis. The Dimertest assay specifically detected 2µg/ml of purified fibrin D-dimer or fibrin D-dimer/fragment E complex added to afibrinogenemic plasma.

US-A-4 090 846 discloses an indirect test for the presence of fibrinogen degradation products in human serum or urine utilizing D and E Fragments coupled to latex carrier products and an antibody specific for the D and E Fragments.

EP-A-0 122 478 discloses a method of preparing monoclonal antibody with specificity for crosslinked fibrin derivatives and an assay procedure using said antibody.

Although the agglutination assay techniques for FDP, including D-dimer, have improved, the stability and sensitivity of these techniques have not been optimized.

The present invention is a simple detection assay for D-dimer using Fragment E attached to a solid phase for direct binding of D-dimer from a biological sample such as plasma, urine, or other tissues or body fluids that may contain the D-dimer Fragment. Accordingly, it is an object of the present invention to provide a direct method to detect D-dimer in a biological sample for diagnosing and monitoring thrombolytic and hypercoagulable states using a novel Fragment E reagent.

This and other objects of the invention will become apparent from the following more detailed description.

The matrix of a blood clot or thrombus is formed by fibrin. Fibrin can be crosslinked by Factor XIIIa to form a stabilized clot. Human crosslinked fibrin is degraded by the enzyme plasmin. The D-dimer/Fragment E complex (DD)E, is the primary soluble plasmin degradation product released from crosslinked fibrin. This complex can be further degraded by plasmin. The initial (DD)E complex contains fragments D-dimer and E₁. Upon further enzymatic digestion, Fragment E₁ is cleaved to Fragment E₂ without loss of the ability to bind to the D-dimer fragment. Digestion of Fragment E₂ to E₃ results in dissociation of the complex with the terminal digestion products of fibrin being Fragments DD and E₃. Fragments E₁ and E₂ have the ability to bind to Fragment DD from crosslinked fibrin but do not bind with the DD-E complex, fibrinogen, or any of the plasmic degradation products of fibrinogen or of non-crosslinked fibrin.

The present invention provides:
An assay method, to directly detect the fibrin breakdown product D-dimer in a biological sample which may contain D-dimer for diagnosing and monitoring thrombolytic and hypercoagulable states, which comprises:
mixing a sample of said biological material which may contain D-dimer with a reagent containing purified Fragment E of human fibrinogen. Further aspects of the invention for which protection is sought are featured in appending claims

Preferably, the purified Fragment E is bound to the latex carrier particles by:
dialysing the purified Fragment E;
concentrating the latex carrier particles;
mixing the dialysed Fragment E with the latex carrier particles;
incubating the mixture; and
separating the resulting latex reagent containing Fragment E bound to the latex carrier particles.

Purified Fragment E can be prepared according to the methods described by Olexa and Budzynski, in Biochemistry 19,991 (1979) and in J. Biol.Chem. 254,4925 (1979). The basic process described in these publications begins with the formation of a fibrin clot from fibrinogen enriched with Factor XIII. The clot is hydrolyzed with plasmin and the resulting digest is centrifuged to remove large clot particles. The supernatant, containing soluble degradation products including the (DD)E complex is then separated on an agarose gel bead column. The separated (DD)E complex is then incubated in a concentrated salt solution to dissociate the D-dimer and E₁ or E₂ fragments. The fragments are then separated by means of an agarose gel bead column and purified Fragment E₁ or E₂ obtained.

The direct binding method to assay for D-dimer of the present invention can be performed in a number of ways. In one embodiment of the invention, purified Fragment E is conjugated to latex carrier particles and an agglutination assay performed. As used within the context of this invention, latex carrier particles include latex polymers which are water insoluble, have a particle size (diameter) in the range of about 0.2 to 1.0 microns, and are inert with respect to immunological reactions. Typical suitable latex carrier particles are those supplied commercially as an aqueous latex suspension usually in concentrations of about 1 to 20% solids. Many types of latex are suitable for use in this invention so long as they meet the criteria listed above. Typical suitable latex carrier polymers are carboxylated polystyrenes, acrylic acid polymers, methacrylic acid polymers, acrylonitrile butadiene styrenes, polyvinyl acetate acrylates, polyvinyl pyridines and vinyl chloride acrylates. The latex concentration in the Fragment E-Latex reagent ranges between 0.5 to 2%.

In a preferred embodiment of the invention, purified Fragment E is conjugated to amidine modified latex beads (Interfacial Dynamics Corporation; 2.4% solids) by mixing equal parts of the amidine modified latex beads (AML) and purified Fragment E to a final concentration of between 0.1 to 10 mg/ml and most preferably, 2 mg/ml. The mixture is incubated overnight at 4° Centigrade, followed by washing in a phosphate buffer solution (PBS). The resulting Fragment E-amidine modified latex reagent is then resuspended in a suitable physiological buffer such as PBS with bovine serum albumin (BSA) or BSA with glycine.

A latex agglutination assay can be performed withthis reagent by combining approximately 25 µl of the Fragment E-AML reagent with approximately 10 µl of plasma, urine or other tissue which may contain D-dimer on a glass slide. After rotating the slide for approximately 2 minutes, observation for agglutination can be made. If agglutination occurs, D-dimer is present in the sample. Agglutination can also be detected using techniques well known in the art such as hemagglutination techniques using microtiter wells, flow cytometry, for example.

In another embodiment of the invention, a non-antibody assay is performed by linking a conjugate to Fragment E, preferably Fragment E₁, but including E₂ and/or E₃. The conjugate-Fragment E reagent is then incubated with a biological sample such as plasma, urine, serum, and then washed to remove excess Fragment E which is not bound to the sample, and the conjugate quantitated for the bound Fragment E-D-dimer production. This method may be performed on a filter device system for detection of D-dimer or the bound versus unbound D-dimer can be separated by molecular exclusion or ion-covalent bond techniques and then detected in the fluid state. Suitable conjugates include peroxidase, urease, beta-galactosidase.

In another embodiment of the invention, a modified ELISA technique can be used to quantitate the concentration of Fragment E₁/E₂, used as the capture molecule for D-dimer. In this method Fragment E₁/E₂ is adsorbed to a solid support. A biological sample such as plasma, urine or other body fluid containing D-dimer is then added for binding to Fragment E₁/E₂. A monoclonal or polyclonal antibody specific for D-dimer is then conjugated to an enzyme such as peroxidase, urease, beta-galactosidase. The conjugate (anti-D-dimer/enzyme) is then allowed to bind to the D-dimer which is directly attached to Fragment E₁/E₂. Addition of a substrate specific for the enzyme used allows for quantitation of D-dimer.

Having generally described the invention, a more complete understanding can be obtained by reference to certain specific examples, which are provided herein for purposes of illustration only and are not intended to be limiting.

### Example 1

### Preparation of the Latex Reagent:

2 mg of purified Fragment E₁ were dialyzed against 3 x 500 ml aliquots of 0.02 M PBS for 6 hours i.e. 3 x 2 hours each. The absorbance (A280) was measured on a Gilford spectrophotometer. The initial reading for a 1 ml volume of dialyzed sample was 3.333 absorbance units. 1 ml (0.489 µ) of amidine modified latex beads (AML), (lot 10-43-57A, Interfacial Dynamics Corporation), was placed into a polypropylene, 1 x 75 mm tube and centrifuged at 3,000 rpm for 15 minutes to pellet the beads. The supernatant was decanted from the pelleted AML beads and 1 ml of dialyzed Fragment E₁ was added. The tube was vortexed gently to resuspend the beads. The Fragment E₁/AML bead mixture was incubated at 4° Centigrade for 12 hours. The tube was then centrifuged to pellet the AML beads. The supernatant was discarded. The absorbance was measured (absorbance 280) at 2.920 absorbance units. 218 µg of Fragment E₁ was bound per ml of AML i.e. 10.9% bound.

### Example 2

### D-dimer Test Procedure:

25 µl of AML-Fragment E₁ latex bead reagent was mixed with 10 µl of either sample (plasma), standard (purified D-dimer), or control (buffer or saline) on a glass slide. The slide was rotated for 2 minutes and observed for any agglutination. The following results were obtained:

| Sample | Lot No. | Reaction |
|---|---|---|
| Saline | 8621 | negative |
| PBS 0.01 M | | negative |
| Purified D-dimer (80 µg/ml) | 101582 | positive |
| American Diagnostic Positive Control | 9203 | positive |
| Stago Positive Control (D-dimer kit) | C211K25 | positive |
| Stago Buffer for Negative Control (D-dimer kit) | C211K25 | negative |
| Wellcome FDP Positive Control (non-specific for D-dimer) | K07691A | negative |
| Wellcome Negative Control (FDP kit) | K07691A | negative |
| Purified Fibrogen (Sigma Bovine Fraction I) | 39B0710 | negative |
| Human Fibrinogen (purified at Ortho) | CH287 | negative |

### Example 3

### Evaluation of Sensitivity of Fragment E₁/AML Reagent:

| Sample | Stago D-dimer Kit Fragment E₁/AML Reagent | |
|---|---|---|
| 80 µg/ml D-dimer | positive | positive |
| 8 µg/ml D-dimer | negative | positive |

## Claims

1. An assay method, to directly detect the fibrin breakdown product D-dimer in a biological sample which may contain D-dimer for diagnosing and monitoring thrombolytic and hypercoaguable states, which comprises:
mixing a sample of said biological material which may contain D-dimer with a reagent containing purified Fragment E of human fibrinogen.

2. The method of claim 1, wherein the reagent comprises the purified Fragment E bound to a solid support.

3. The method of claim 2, wherein the solid support comprises latex carrier particles.

4. The method of claim 3, wherein the purified Fragment E is bound to the latex carrier particles by:
dialysing the purified Fragment E;
concentrating the latex carrier particles;
mixing the dialysed Fragment E with the latex carrier particles;
incubating the mixture; and
separating the resulting latex reagent containing Fragment E bound to the latex carrier particles.

5. The method of claim 3 or claim 4, wherein the latex carrier particles comprise amidine modified latex beads.

6. The method of any one of claims 3 to 5, wherein the mixture of the sample of the biological fluid and the reagent containing Fragment E bound to latex carrier particles is observed for agglutination.

7. The method of any one of claims 2 to 5, which comprises the steps of; adding, to the mixture of the sample of biological material and the reagent containing the purified Fragment E bound to a solid support, an antibody specific for D-dimer conjugated to a label; and detecting label bound to the solid support.

8. The method of claim 7, wherein the label is an enzyme and the detecting step comprises adding a substrate specific for the enzyme.

9. The method of claim 1, wherein the reagent comprises a conjugate of purified Fragment E and a protein.

10. The method of claim 9, wherein the protein is peroxidase.

11. The method of claim 9 or claim 10, wherein the mixture of the sample of biological material and the reagent is treated to separate conjugate bound to D-dimer from unbound conjugate and the amount of bound or unbound conjugate is determined.

12. The method of any one of claims 1 to 11, wherein the final concentration of the Fragment E in the reagent is 0.1 mg/ml to 10 mg/ml, preferably about 2 mg/ml.

13. The method of any of claims 1 to 12, wherein the Fragment E comprises Fragment E₁.

## Patentansprüche

1. Testverfahren zum direkten Nachweis des Fibrinabbauproduktes D-Dimer in einer biologischen Probe, die D-Dimer enthalten kann, zur Diagnose und Überwachung thrombolytischer und hyperkoagulabler Zustände, das umfaßt:
Mischen einer Probe des biologischen Materials, das D-Dimer enthalten kann, mit einem Reagenz, das gereinigtes Fragment E von Humanfibrinogen enthält.

2. Verfahren nach Anspruch 1, bei dem das Reagenz das gereinigte Fragment E gebunden an einen festen Träger umfaßt.

3. Verfahren nach Anspruch 2, bei dem der feste Träger Latexträgerpartikel umfaßt.

4. Verfahren nach Anspruch 3, bei dem das gereinigte Fragment E dadurch an die Latexträgerpartikel gebunden wird, daß man:
das gereinigte Fragment E dialysiert;
die Latexträgerpartikel konzentriert;
das dialysierte Fragment E mit den Latexträgerpartikeln mischt;
das Gemisch inkubiert; und
das erhaltene Latexreagenz, das an die Latexträgerpartikel gebundenes Fragment E enthält, abtrennt.

5. Verfahren nach Anspruch 3 oder Anspruch 4, bei dem die Latexträgerpartikel amidinmodifizierte Latexbeads umfassen.

6. Verfahren nach einem der Ansprüche 3 bis 5, bei dem das Gemisch der Probe der biologischen Flüssigkeit und des Reagenzes, das Fragment E gebunden an Latexträgerpartikel enthält, auf Agglutination untersucht wird.

7. Verfahren nach einem der Ansprüche 2 bis 5, das die Schritte umfaßt: Zugeben eines für D-Dimer spezifischen Antikörpers, der an einen Marker konjugiert ist, zu dem Gemisch der Probe aus biologischem Material und dem Reagenz, das das gereinigte Fragment E gebunden an einen festen Träger enthält, und Nachweisen von an den festen Träger gebundenem Marker.

8. Verfahren nach Anspruch 7, bei dem der Marker ein Enzym ist und der Nachweisschritt die Zugabe eines für das Enzym spezifischen Substrats umfaßt.

9. Verfahren nach Anspruch 1, bei dem das Reagenz ein Konjugat von gereinigtem Fragment E und einem Protein umfaßt.

10. Verfahren nach Anspruch 9, bei dem das Protein Peroxidase ist.

11. Verfahren nach Anspruch 9 oder 10, bei dem das Gemisch aus der Probe von biologischem Material und dem Reagenz behandelt wird, um an D-Dimer gebundenes Konjugat von ungebundenem Konjugat abzutrennen und die Menge von gebundenem oder ungebundenem Konjugat bestimmt wird.

12. Verfahren nach einem der Ansprüche 1 bis 11, bei dem die Endkonzentration des Fragments E in dem Reagenz 0,1 mg/ml bis 10 mg/ml, vorzugsweise etwa 2 mg/ml beträgt.

13. Verfahren nach einem der Ansprüche 1 bis 12, bei dem das Fragment E Fragment E₁ umfaßt.

## Revendications

1. Procédé d'essai pour détecter directement le produit dimère D de dégradation de la fibrine dans un échantillon biologique susceptible de contenir du dimère D, pour le diagnostic et le contrôle d'états thrombolytiques et hypercoagulables, comprenant:
le mélange d'un échantillon dudit matériel biologique, susceptible de contenir du dimère D, avec un réactif contenant du fragment E purifié de fibrinogène humain.

2. Procédé de la revendication 1, dans lequel le réactif comprend le fragment E purifié lié à sur un support solide.

3. Procédé de la revendication 2, dans lequel le support solide comprend des particules de véhicule en latex.

4. Procédé de la revendication 3, dans lequel le fragment E purifié est lié aux particules de véhicule en latex par:
dialyse du fragment E purifié;
concentration des particules de véhicule en latex;
mélange du fragment E dialysé avec les particules de véhicule en latex;
incubation du mélange; et
séparation du réactif de latex résultant, contenant du fragment E lié
aux particules de véhicule en latex.

5. Procédé de la revendication 3 ou 4, dans lequel les particules de véhicules en latex comprennent des billes de latex modifiées par de l'amidine.

6. Procédé de l'une quelconque des revendications 3 à 5, dans lequel on examine le mélange de l'échantillon du liquide biologique et du réactif contenant du fragment E lié aux particules de véhicules en latex, pour déterminer l'agglutination.

7. Procédé de l'une quelconque des revendications 2 à 5, comprenant les étapes suivantes: addition, au mélange de l'échantillon de matériel biologique et du réactif contenant le fragment E lié à un support solide, d'un anticorps spécifique du dimère D conjugué à un marqueur; et détection du marqueur lié au support solide.

8. Procédé de la revendication 7, dans lequel le marqueur est une enzyme et l'étape de détection comprend l'addition d'un substrat spécifique pour l'enzyme.

9. Procédé de la revendication 1, dans lequel le réactif comprend un conjugué de fragment E purifié et d'une protéine.

10. Procédé de la revendication 9, dans lequel la protéine est une peroxydase.

11. Procédé de la revendication 9 ou 10, dans lequel on traite le mélange du matériel biologique et du réactif afin de séparer le conjugué lié au dimère D d'avec le conjugué non lié, et on détermine la quantité de conjugué lié ou non lié.

12. Procédé de l'une quelconque des revendications 1 à 11, dans lequel la concentration finale du fragment E dans le réactif va de 0,1 à 10 mg/ml, et de préférence est d'environ 2 mg/ml.

13. Procédé selon l'une quelconque des revendications 1 à 12, dans lequel le fragment E comprend le fragment E₁.
